# EUROPEAN PATENT SPECIFICATION

(11) **EP 3 846 773 B1**
(45) Date of publication and mention of the grant of the patent: **20.12.2023**
(21) Application number: 19758982.3
(22) Date of filing: 28.08.2019
(51) Int. Cl.: A61K 8/66, A61Q 19/08, A61K 8/9789, A23L 3/3571, A23B 4/22, C12N 9/02, C12N 15/82

(54) **INDUSTRIAL APPLICATIONS OF PLANT CELL EXTRACTS COMPRISING SOD ENZYMES OF EXTREMOPHILIC MICRO-ORGANISMS**
INDUSTRIELLE ANWENDUNGEN VON PFLANZENZELLEXTRAKTEN MIT SOD-ENZYMEN VON EXTREMOPHILEN MIKROORGANISMEN
APPLICATIONS INDUSTRIELLES D'EXTRAITS DE CELLULES VÉGÉTALES COMPRENANT DES ENZYMES SOD DE MICRO-ORGANISMES EXTRÉMOPHILES

(30) Priority: 03.09.2018 IT 201800008320
(43) Date of publication of application: 14.07.2021
(73) Proprietor: Arterra Bioscience S.p.A., 80142 Napoli (IT)
(72) Inventor: ARCIELLO, Stefania, 80142 Napoli (IT); APONE, Fabio, 80142 Napoli (IT); COLUCCI, Maria Gabriella, 80142 Napoli (IT); PALMIERI, Gianna, 80131 Napoli (IT); COCCA, Ennio, 80131 Napoli (IT)
(74) Representative: Botti & Ferrari S.p.A.
(86) International application number: PCT/EP2019/072912
(87) International publication number: WO 2020/048836

(56) References cited:
- EP-A2- 0 359 617
- EP-A2- 0 952 224
- US-A- 5 538 878
- ALLEN R D: "DISSECTION OF OXIDATIVE STRESS TOLERANCE USING TRANSGENIC PLANTS", PLANT PHYSIOLOGY, AMERICAN SOCIETY OF PLANT PHYSIOLOGISTS, ROCKVILLE, MD, USA, vol. 107, 1 January 1995 (1995-01-01), pages 1049-1054, XP002072721, ISSN: 0032-0889
- DATABASE WPI Week 201619 Thomson Scientific, London, GB; AN 2016-02257W XP002791229, LIU X, QUIN J, YUE Y: "Superoxide dismutase used in skin care products, is obtained air born bacteria, bacterial leaf streak and thermophilic solfataricus", & CN 105 112 379 A (SHANGHAI JULANG BIOTECHNOLOGY CO LTD) 2 December 2015 (2015-12-02)
- RAFFAELE CANNIO ET AL: "A superoxide dismutase from the archaeon Sulfolobus solfataricus is an extracellular enzyme and prevents the deactivation by superoxide of cell-bound proteins : A superoxide dismutase secreted by S.?solfataricus", EUROPEAN JOURNAL OF BIOCHEMISTRY, vol. 267, no. 1, 1 January 2000 (2000-01-01), pages 235-243, XP055587068, GB ISSN: 0014-2956, DOI: 10.1046/j.1432-1327.2000.00997.x
- Hee-Jin Lee ET AL: "An efficient method for the expression and reconstitution of thermostable Mn/Fe superoxide dismutase from Aeropyrum pernix K1", Journal of microbiology and biotechnology, 1 April 2010 (2010-04-01), pages 727-731, XP055587070, Korea (South) DOI: 10.4014/jmb.0911.11008 Retrieved from the Internet: URL:http://www.jmb.or.kr/submission/Journa l/020/JMB020-04-12_FDOC_1.pdf
- Lye Meng Markillie ET AL: "Targeted Mutagenesis by Duplication Insertion in the Radioresistant Bacterium Deinococcus radiodurans: Radiation Sensitivities of Catalase (katA) and Superoxide Dismutase (sodA) Mutants", Journal of Bacteriology, 15 January 1999 (1999-01-15), pages 666-669, XP055587101, United States Retrieved from the Internet: URL:https://jb.asm.org/content/jb/181/2/66 6.full.pdf
- DATABASE WPI Week 201432 1 August 2014 (2014-08-01) Thomson Scientific, London, GB; AN 2014-J22401 XP002791230, MA X: "Anti-aging healthcare food comprises superoxide dismutase, spirulina, aromatic oil, grape seed and catechin", & CN 103 637 192 A (WEIHAI WUGU YIJIAN FOOD CO LTD) 19 March 2014 (2014-03-19)
- ANNALISA TITO ET AL: "A tomato stem cell extract, containing antioxidant compounds and metal chelating factors, protects skin cells from heavy metal-induced damages : A tomato cell extract protects skin from heavy metals", INTERNATIONAL JOURNAL OF COSMETIC SCIENCE., vol. 33, no. 6, 1 December 2011 (2011-12-01), pages 543-552, XP055587056, NL ISSN: 0142-5463, DOI: 10.1111/j.1468-2494.2011.00668.x
- MAS-CHAMBERLIN C ET AL: "HEAT- AND UV-STABLE COSMETIC ENZYMES FROM DEEP SEA BACTERIA", COSMETICS & TOILETRIES, ALLURED PUBLISHING CORP, US, vol. 117, no. 4, 1 April 2002 (2002-04-01) , pages 22, 24-28, 30, XP008016986, ISSN: 0361-4387
- J.K. DONNELLY ET AL: "Superoxide dismutases in foods. A review", FOOD CHEMISTRY, vol. 33, no. 4, 1 January 1989 (1989-01-01), pages 243-270, XP055587052, NL ISSN: 0308-8146, DOI: 10.1016/0308-8146(89)90036-8

## Description

### Field of invention

The present invention relates, in general, to the industrial use of extracts derived from plant cell cultures comprising enzymes of extremophilic organisms, and the related compositions and formulations comprising them for use in dermocosmetic, biomedical, nutraceutical and food sectors.

### State of art

It is known that the oxidation of proteins, DNA, lipids and other macromolecules, which form living cells, is a chemical process triggered by an excess of reactive oxygen species (ROS) or other free radicals, derived from endogenous (metabolism and other cellular processes) and exogenous sources (exposure to cigarette smoke, ozone, ionizing radiations, heavy metals and UV radiations). The main ROS of physiological importance are: the superoxide anion (O₂⁻), the radical hydroxyl (OH⁻) and the hydrogen peroxide (H₂O₂). At moderate concentrations, ROS participate in the physiological processes of the cell, but at higher levels they can alter the structure of DNA and irreversibly modify proteins and lipids. In particular, membrane lipids are the molecules most exposed to the extra-cellular environment and, therefore, are the first to be attacked by ROS which compromise the structural and functional integrity of lipids and the whole cell, by triggering a process of "peroxidation", (Ayala et al., 2014).

Due to the ROS dangerousness , the cell has developed protection mechanisms, including "*scavengers*" mediated by antioxidant enzymes, being able to convert the ROS into molecular oxygen (O₂) and water (H₂O). Nevertheless, when the ROS levels exceed the cell capacity to neutralize them through protection mechanisms, a condition called "oxidative stress" occurs, in which the reactivity of the free radicals prevails, often caused by either extrinsic or external factors, including exposure to cigarette smoke, ozone, ionizing radiation, heavy metals (copper, iron, cadmium, lead and mercury) and UV radiations.The superoxide anion (O₂⁻) is the main free radical produced during oxidative stress conditions and can be converted by the enzyme SuperOxide Dismutase (SOD) into H₂O₂, which is in turn transformed into H₂O and O₂ by the enzyme catalase. Superoxide dismutases, together with catalases and glutathione peroxidases, constitute the so-called "first line defense system" from the oxidative stress (Ighodaro and Akinloye, 2017).

It is also known that superoxide dismutases are antioxidant enzymes of the "metalloprotein" family and, therefore, require a metal ion as a cofactor to carry out their enzymatic functions.

Depending on the associated metal ion, superoxide dismutases are classified into Fe-SOD (present in prokaryotes and chloroplasts of some plants), Mn-SOD (present in prokaryotes and eukaryotic mitochondria), and Cu/Zn-SOD (present in eukaryotes and localized in the cytosol, peroxisomes and chloroplasts of plants).

Therefore, superoxide dismutases are found in all living organisms and are particularly concentrated in brain, liver, heart, kidney and red blood cells in humans. In human cells, Cu/Zn-SODs are located in the cytosol, while Mn-SODs in the mitochondria, although extracellular isoforms exist in the tissues interstitial spaces and in extracellular fluids such as plasma, lymph and synovial fluids. The association between SOD deficiency and a significant number of diseases has been observed both in animals and in humans.

In fact, the superoxide dismutases, as antioxidant enzymes, are important for the prevention of neurodegenerative diseases such as Alzheimer's, Parkinson's and atherosclerosis, because they counteract the development of reactive oxygen species. Indeed, it is known that the SOD levels decrease significantly with the cell age, while the free radicals progressively increase (Valgimigli et al, 2015). It has been suggested that an adequate SOD supplementation could slow down the entire cellular aging process, protect the immune system and significantly reduce the possible occurrence of diseases.

The oxidation phenomenon also affects the conservation of food products.

In fact, it is known that the oxidation of products such as meat and fish leads to a rapid deterioration of the appearance, taste and nutritional value of the product; in extreme situations, an incorrect storage of meat can lead to food poisoning due to the high levels of histamine in the food. Indeed, histamine is highly thermostable and is not destroyed by normal cooking temperatures; as a consequence, even if subsequently cooked, improperly preserved meat can contain high levels of histamine (considered as an early indicator of decomposition) and can compromise the health of the consumer.

In particular, appearance and color of red meat fish, such as tuna, are important parameters for the qualitative evaluation of the food and can determine their commercial value. Consumers are generally attracted by the bright red color given by oximyoglobin, which is generated when oxygen binds heme group of myoglobin. Instead, the brown-hazel coloration, caused by a self-oxidation process of oxymyoglobin to metamyoglobin, is typical of a product that is no longer fresh.

It is also known another process of oxidation of food products, in particular of oils and unsaturated fats, that is the phenomenon of rancidity. This is due to a series of lipid oxidation reactions that lead to the formation of short chain fatty acids, such as propionic acid and butyric acid, resulting in the production of an unpleasant odor and an altered taste.

It is known the the use of antioxidants to counteract the aforementioned oxidation phenomena, such as vitamin C, vitamin E and other compounds of synthetic origin, such as t-butyl-4-hydroxyanisole, known as BHA 20. Although being the most effective, BHA cannot be used in large quantities as it is a harmful compound that can cause several side effects, while vitamins are not very stable over time and are subject to hydrolytic processes caused by high temperature or high salt concentration conditions (Donnelly et al., 1989).

It is also known the use of superoxide dismutase in the dermo-cosmetic, biomedical and nutraceutical as well as food sectors, in which compounds capable of neutralizing free radicals formation are strongly needed..

In particular, the superoxide dismutases used in industrial sectors usually derive from mesophilic organisms and, therefore, they are stable and active in temperature conditions between 37 and 50°C, salinity 1%, neutral pH 7.0-7.5. Thus, these enzymes are not active in temperature, salinity and pH conditions harsher than those mentioned above, and under conditions of exposure to UV radiation, and this limits their use at industrial level.

For example, the use of SOD in sunscreen products for skin presents the problem of poor UV and temperature stability; the enzyme added in a nutraceutical product presents problems related to the structural stability due to the extremely acid pH in the stomach environment; finally, products meant for the food market are often characterized by high concentrations of salts which can also negatively influence the enzymatic activity of SODs.

Furthermore, it is known that the enzymes produced by processes of isolation and purification from bacteria can be exposed to possible contamination by toxic or allergenic compounds of bacterial origin.

The problem underlying the present invention is that of providing a composition comprising superoxide dismutases that are stable and active in a wide range of temperature, salinity and pH and/or in the presence of UV radiation, and that are free of any contaminant, toxic substance, allergenic or bacteria derived compound .

### Summary of the invention

The aforesaid technical problem is solved, according to the present invention, by providing a composition with antioxidant activity comprising a first dry extract derived from plant cell cultures comprising superoxide dismutase of *Sulfolobus solfataricus* expressed in said plant cells, a second dry extract derived from plant cell cultures comprising superoxide dismutase of *Aeropyrum pernix* expressed in said plant cells, and a third dry extract derived from plant cell cultures comprising superoxide dismutase of *Deinococcus* radiodurans expressed in said plant cells.

Preferably, the plant cell cultures are cultures of cells belonging to *Solanum lycopersicum* species.

Preferably, the composition contains, for one part by weight of said dry extract comprising superoxide dismutase of *Sulfolobus solfataricus*, from 0.01 to 100, preferably 0.5-2, parts by weight of said dry extract comprising superoxide dismutase of *Aeropyrum pernix*, and, independently, from 0.01 to 100, preferably 0.5-2, parts by weight of said dry extract comprising superoxide dismutase of *Deinococcus radiodurans.*

Preferably, the composition contains, for one part by weight of said dry extract comprising superoxide dismutase of *Sulfolobus solfataricus*, one part by weight of said dry extract comprising superoxide dismutase of *Aeropyrum pernix* and one part by weight of said dry extract comprising superoxide dismutase of *Deinococcus radiodurans.*

Preferably, the composition further comprises at least one hydrophilic solvent selected from the group comprising water, saline aqueous solutions and organic solvents.

In an aspect thereof, the present invention refers to the cosmetic use of a composition as defined above.

In another aspect thereof, the present invention refers to the use of a composition as defined above as food preservative.

The present invention also relates to a cosmetic formulation for topical application, comprising a composition as defined above, at least one cosmetically active substance and a cosmetically acceptable vehicle.

Preferably, the above-mentioned formulation is in the form of a gel, a cream or a lotion.

In an aspect thereof, the present invention also relates to a food or nutraceutical product comprising a composition as defined above.

Advantageously, the composition of the present invention comprises plant cell culture extracts which comprise superoxide dismutases derived from extremophilic microorganisms, which are stable and active in wide ranges of temperature, salinity and pH and/or in the presence of UV radiation. The use of the three extracts in a mixture represents an advantage compared to the use of the single preparations, since the presence in the mixture of three extremophile SOD, each of which has different characteristics and high catalytic performances in different physical conditions, allows to cover a wide range of applications that require an antioxidant action in conditions of high temperature, high salinity, low pH or high doses of UV rays.

This advantageous feature makes the present composition suitable for the use in the dermocosmetic sector, as it is able to exert a stable and long-lasting antioxidant action even after prolonged exposure to UV rays and/or heat.

Moreover, it is suitable for the use in the field of food preservation, because the superoxide dismutases contained in the extracts of the present invention remain stable and active even in the presence of high concentrations of salts and/or pH values even very far from neutrality (e.g. at pH between 3 and 10), significantly counteracting the process of food oxidation and the appearance of alterations of the products related to it.

A further advantage is the fact that the extracts contained in the composition of the present invention are obtained from *in vitro* plant cell cultures and, therefore, are free of any allergenic substance and/or irritants of bacterial origin as well as toxic contaminants (for example, pesticides and/or fertilizers) and environmental pathogen agents, because plant cell cultures grow in controlled conditions in the laboratory.

In one embodiment, plant cell cultures are cultures of cells belonging to *Solanum lycopersicum* species (tomato).

Advantageously, the extracts of the cell cultures from this specific plant species are particularly effective in protecting skin cells from the toxic action of heavy metals and in inducing endogenous collagen production, due the presence of several beneficial secondary metabolites (Tito et al., 2011). Furthermore, the extracts derived from tomato cells do not contain substances that are potentially allergenic or toxic to the cells, such as the solanine and tomatine alkaloids which, instead, are present in the leaves and fruits of the tomato plants.

### Brief description of the figures

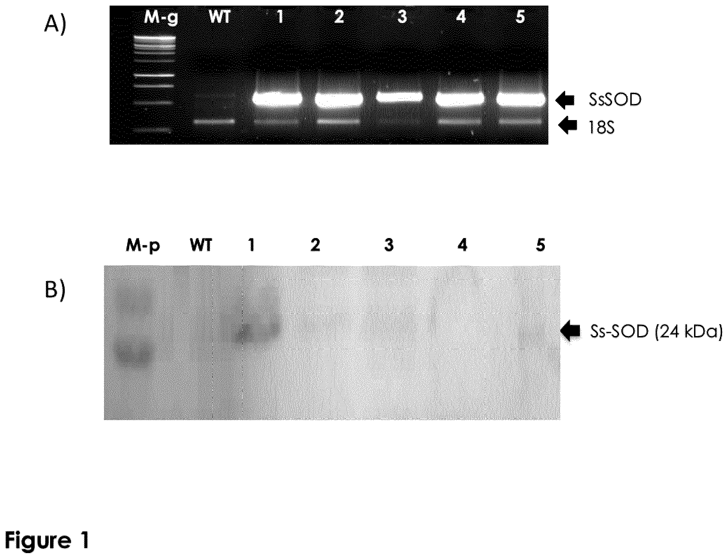

**Figure 1A** represents an image of a gel after electrophoresis of the amplified SOD gene of *Sulfolobus solfataricus* (SsSOD) in untransformed tomato cells (WT) and transformed lines (1-5); gene expression was normalized to the reference standard gene 18S ribosomalRNA . M-g: Genes marker containing DNA fragments of known molecular weight.

**Figure 1B** represents an image of a polyacrylamide gel in the presence of sodium dodecyl sulfate (SDS-PAGE), on which a Western-Blot analysis of the untransformed tomato cell extracts (WT) and the transformed tomato cell lines was carried out (1-5). About 20 µg of total protein was separated on a 18% SDS-PAGE gel, transferred to a membrane and incubated with an anti-SOD primary antibody (1: 1000). M-p: Proteins marker (Precision Plus Protein Standard, Bio-Rad).

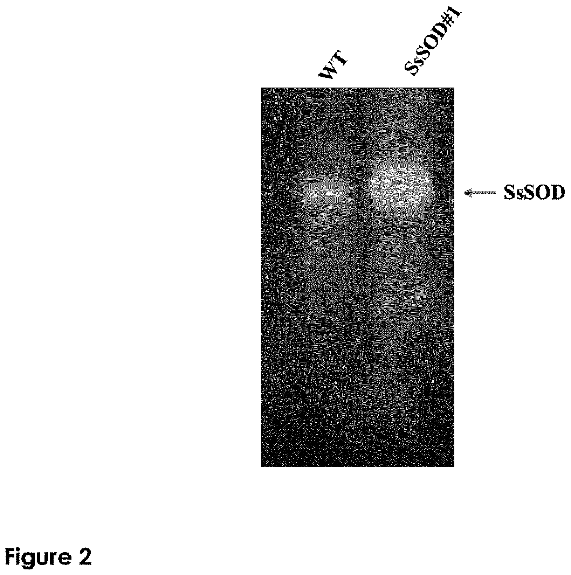

**Figure 2** represents an image of a polyacrylamide gel after electrophoresis in non-denaturing conditions (native-PAGE) of the extracts of untransformed cell cultures (WT) and cell cultures of line 1transformed with the gene coding for SsSOD (SsSOD#1). For each lane of the gel, 15 µg of total proteins was analyzed and the activity was revealed by staining for SOD activity.

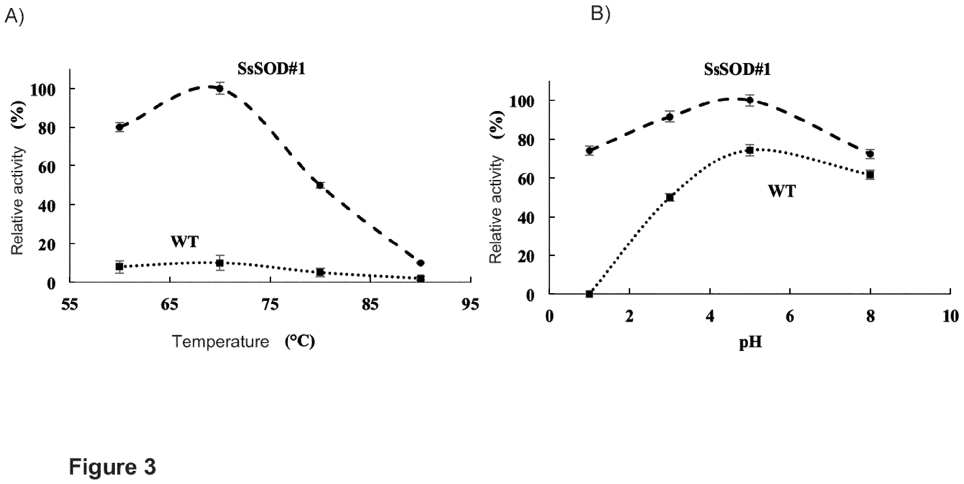

**Figures 3A and 3B** respectively show a graph of the antioxidant activity as a function of temperature (A) and pH (B) of the extract of untransformed tomato cell cultures (WT) and the tomato cell extract from line 1 (SsSOD#1). The enzymatic activity values, both of the WT extract and the SsSOD#1 line extract , have been expressed with respect to the highest enzymatic activity corresponding to 100%.

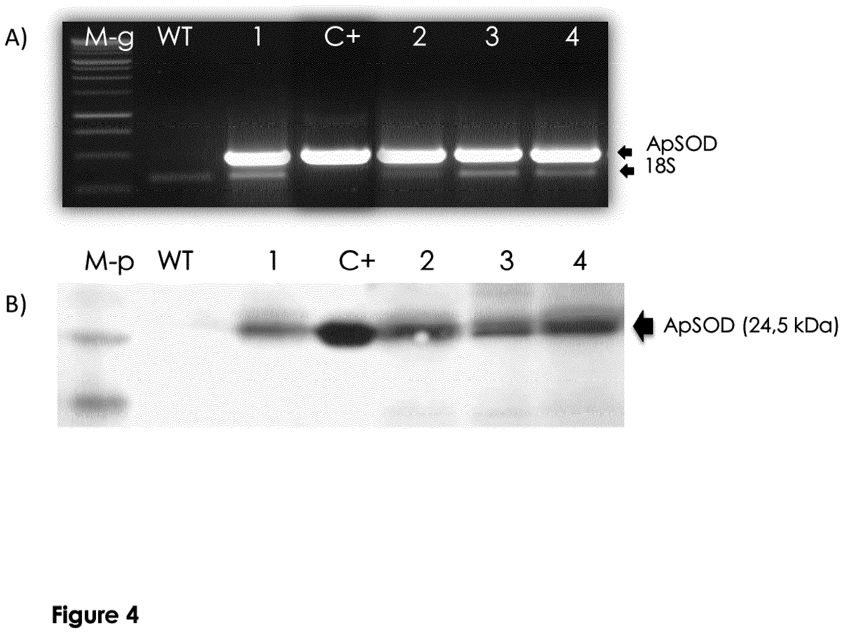

**Figure 4A** represents an image of a gel after electrophoresis of *Aeropyrum pemix* SOD gene amplifications (ApSOD) in untransformed tomato cells (WT) and the transformed tomato cell lines (1-4); the gene expression was normalized to the standard gene 18S. M-g: Genes marker containing DNA fragments of known molecular weight; C+: positive control represented by a plasmid containing the ApSOD gene.
**Figure 4B** represents an image of a sodium dodecyl sulfate polyacrylamide gel (SDS-PAGE) on which a Western-Blot analysis of the extracts of untransformed tomato cells (WT) and the transformed lines was carried out (1-4). Approximately 20µg of total protein was separated on 18% SDS-PAGE, transferred to a blot and incubated with an anti-SOD primary antibody (1:1000). M-p: Protein marker (Precision Plus Protein Standard, Bio-Rad); C+: SOD protein expressed and purified from bacteria.

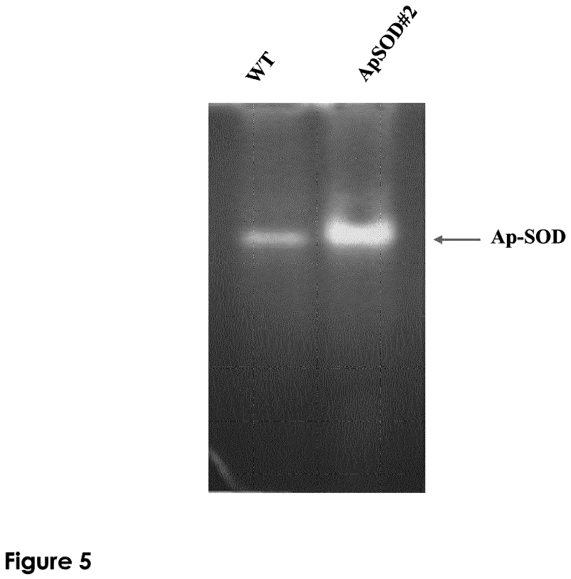

**Figure 5** shows an image of a polyacrylamide gel after electrophoresis in non-denaturing conditions (native-PAGE) of the extracts of the untransformed tomato cell cultures (WT) and cell cultures of line 2 (ApSOD#2), transformed with the gene encoding for ApSOD. For each lane, 15 µg of total protein was analyzed, and SOD activity was revealed by staining.

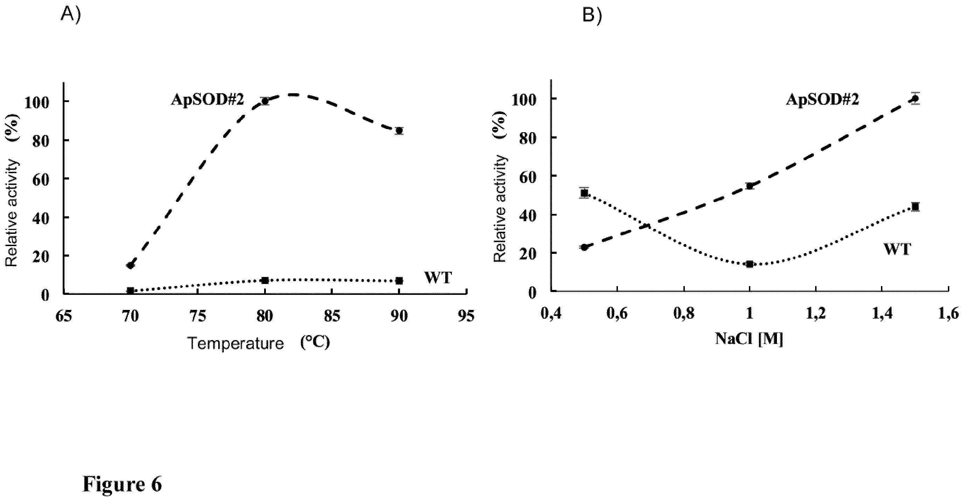

**Figures 6A and 6B** show a graph of the antioxidant activity as a function of temperature (A) and NaCl concentration (B) of the extracts of untransformed tomato cell cultures (WT) and tomato cells of line 2 ( ApSOD#2), respectively. The values of enzymatic activity, both for the WT extract and ApSOD#2, have been expressed with respect to the highest enzymatic activity corresponding to 100%.

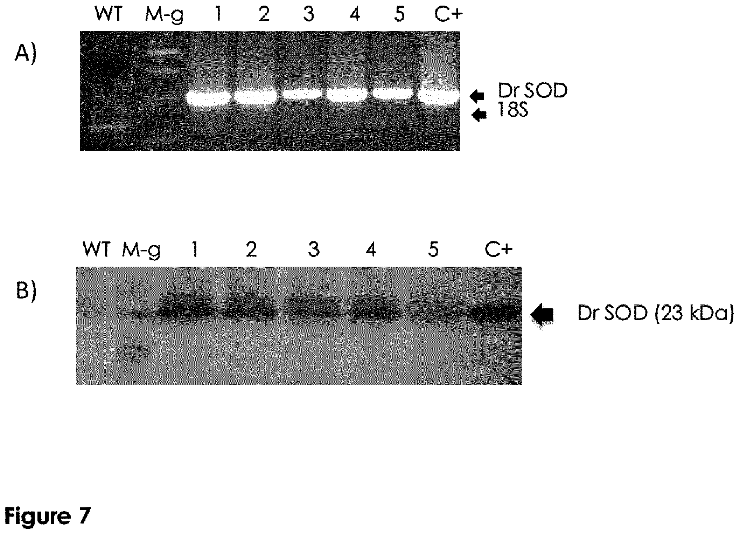

**Figure 7A** represents an image of a gel after electrophoresis of *Deinococcus radiodurans* SOD gene (DrSOD) amplifications in the extract of the untransformed tomato cell culture (WT) and in the extract of the transformed tomato lines (1-5). The gene expression was normalized with respect to the standard gene 18S. M-g: Genes marker containing DNA fragments of known molecular weight; C+: positive control represented by a plasmid containing the gene of DrSOD.
**Figure 7B** represents an image of a sodium dodecyl sulfate polyacrylamide gel (SDS-PAGE), on which a Western-Blot analysis of the extracts of untransformed tomato cells (WT) and the transformed lines was carried out (1-4). Approximately 20µg of total proteins was separated on 18% SDS-PAGE, transferred to a blot and incubated with an anti-SOD primary antibody (1:1000). M-p: Protein marker (Precision Plus Protein Standard, Bio-Rad); C+: SOD protein expressed and purified from bacteria.

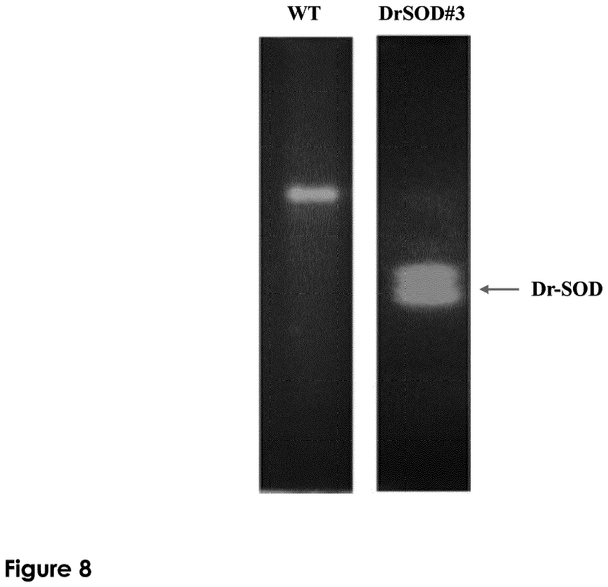

**Figure 8** represents an image of a polyacrylamide gel after electrophoresis in non-denaturing conditions (native-PAGE) of the untransformed tomato cell culture extracts (WT) and the tomato cell culture extracts of line 3 (DrSOD#3), transformed with the gene coding for the DrSOD. For each lane, 15 µg of total proteins was analyzed, and SOD activity was revealed by staining.

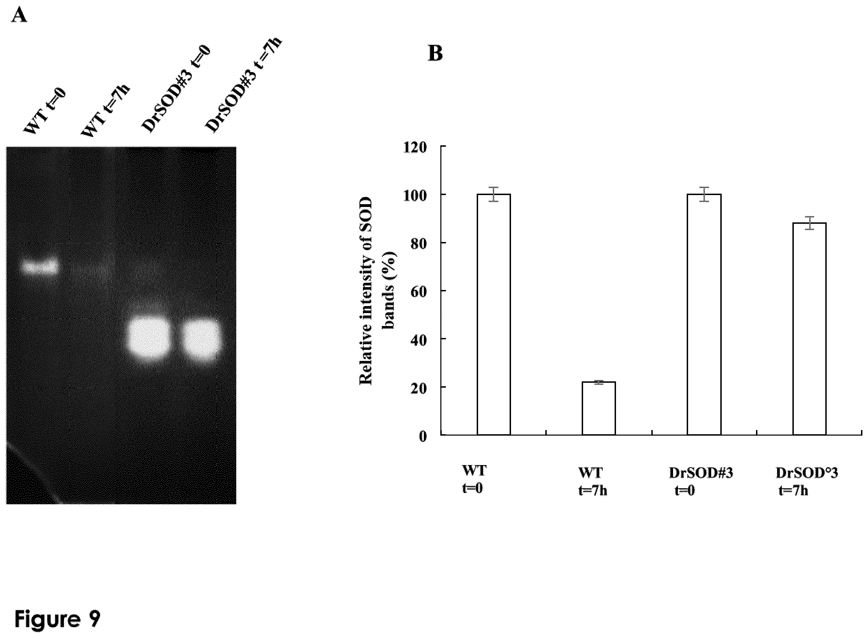

**Figure 9A** represents an image of a polyacrylamide gel after electrophoresis in non-denaturing conditions (native-PAGE) showing the effect of the exposure to UVB rays for 7 hours on the protein extracts comprising DrSOD. The protein bands and SOD activity were revealed by staining before the treatment with UV (t=0) and after the treatment with UV for 7 hours (t=7 hours).
**Figure 9B** shows a histogram graph relating to a densitometric analysis of the band intensity of the SOD activity of the gel shown in Figure 9A (the signal intensity of the SOD activity band at t=7h was reported in a percentage value with respect to the signal intensity of each SOD activity band at t=0, set as 100%).

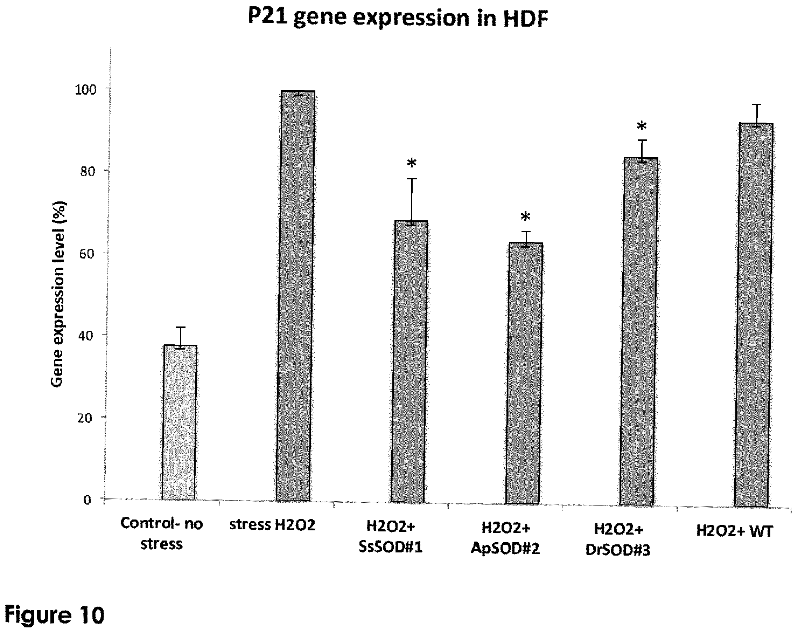

**Figure 10** illustrates a bar graph of the expression levels of p21 gene in human fibroblasts (HDF). HDF cells were treated with the WT tomato cell extract and with those derived from the 3 transgenic lines (SsSOD#1, ApSOD#2, DrSOD#3), containing the extremophilic SOD, at a concentration of 0.002%. After the cells were subjected to oxidative stress in the presence of H₂O₂ hydrogen peroxide (100 µM) for 2 days, the expression levels of the p21 gene were determined and compared with that of the HDF cells treated only with hydrogen peroxide, set as 100 %. The asterisks indicate the significant differences with respect to the control.

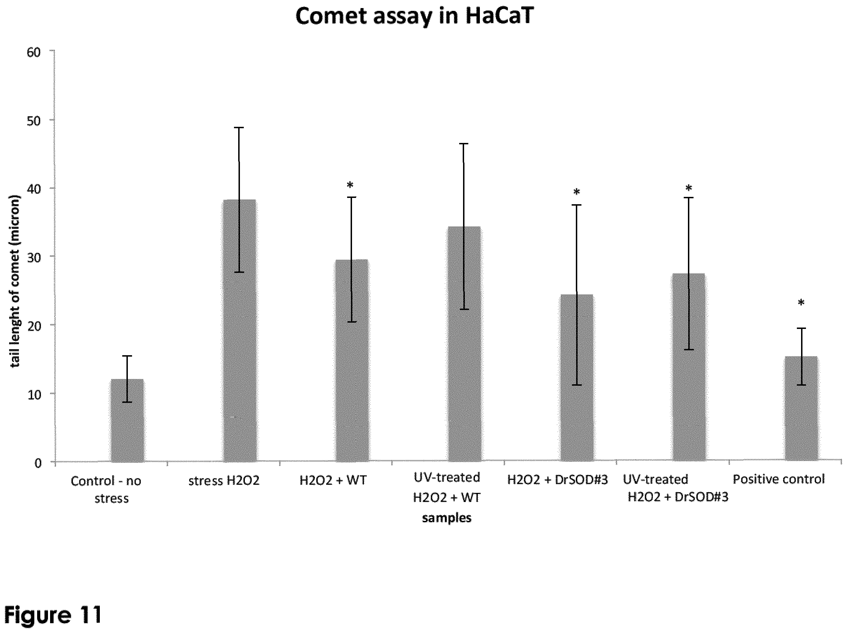

**Figure 11** shows a bar graph of the average length of the cellular nuclei comet tails, caused by the exposure to hydrogen peroxide of human keratinocytes (HaCaT) treated with the extract of WT tomato cells and the extract of DrSOD transformed cells (DrSOD#3), according to the assay "Comet". Both the WT cell samples and those of the transgenic lines were treated for 7 hours with UV light, and the length of the nuclear tails of the different samples was compared. As a positive assay control, a water-soluble extract of *Dolichos* cell cultures, already characterized by Arterra for its ability to protect against H₂O₂ damage, was used (Bimonte et al., 2013).

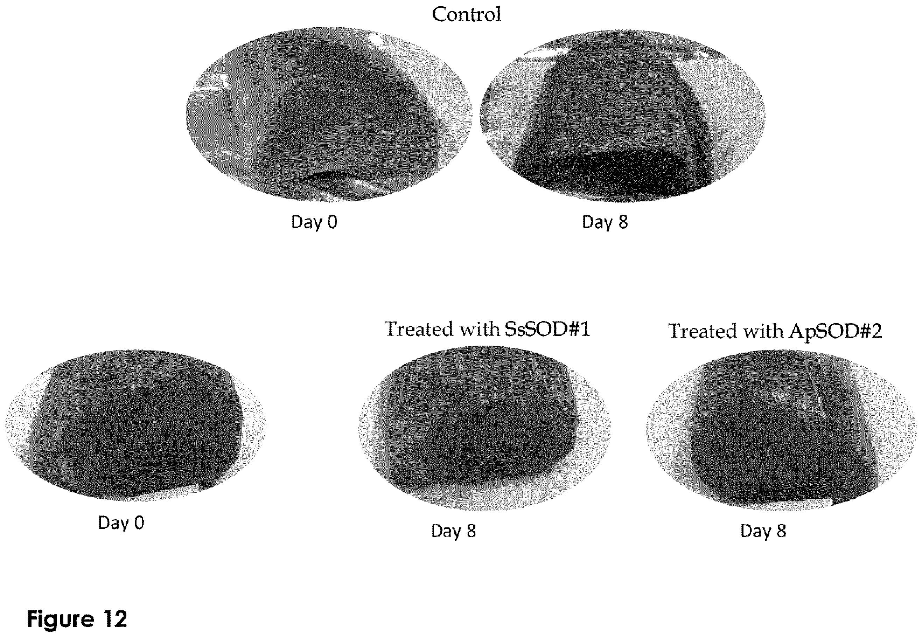

**Figure 12** illustrates the photos of tuna slices treated with the WT tomato cell extract (control) and extracts derived from cells expressing the SsSOD (SsSOD#1) and ApSOD (ApSOD#2), at time 0 and after 8 days.

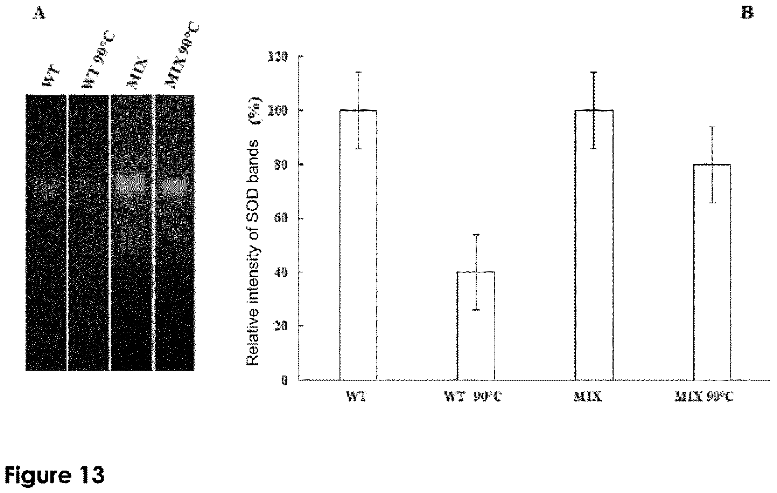

**Figure 13** shows in: (A) an image of a polyacrylamide gel after electrophoresis in non-denaturing conditions (native-PAGE) that shows the effect of the treatment at the temperature of 90°C on the control extracts from untransformed tomato cells (WT) and on the mixture (MIX) consisting of the extracts from tomato cells comprising SsSOD, ApSOD and DrSOD. The bands on the gel and SOD activity were revealed by staining, both before and after the treatment with heat at 90°C; B) a histogram graph relating to the densitometric analysis of the intensity of the SOD activity bands of the gel shown in A (the signal intensity of the SOD activity band at heat treatment was reported in a percentage value with respect to the intensity of the signal of each SOD activity band of the untreated samples, corresponding to 100%).

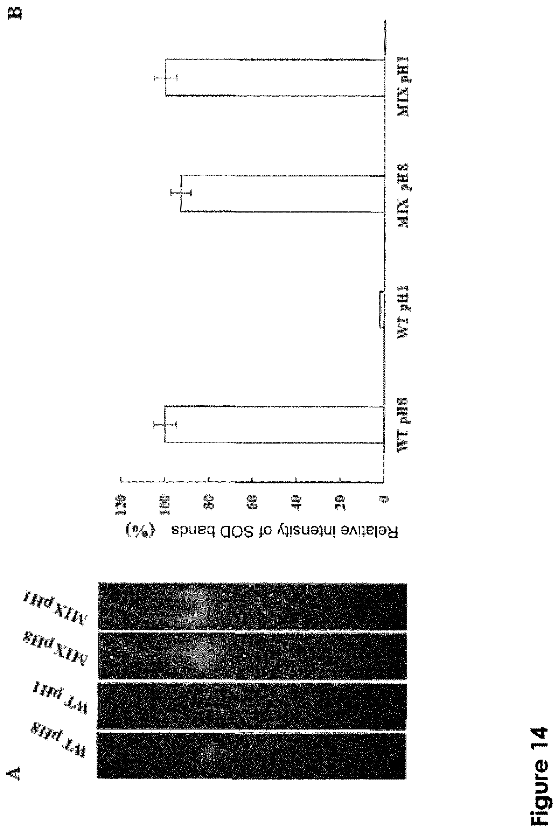

**Figure 14** shows in: (A) an image of a polyacrylamide gel after electrophoresis in non-denaturing conditions (native-PAGE) that shows the effect of the treatment at pH 1.0 on the protein extracts from untransformed tomato cells (WT) and on the mixture (MIX), consisting of the extracts from tomato cells containing the SsSOD, ApSOD and DrSOD. The bands of SOD activity were revealed by staining; (B) a histogram graph relating to the densitometric analysis of the intensity of the SOD activity bands of the gel shown in A (the signal intensity of the SOD activity band at pH 1 was reported as percentage value with respect to the intensity of the signal of each SOD activity band at pH 8, corresponding to 100%).

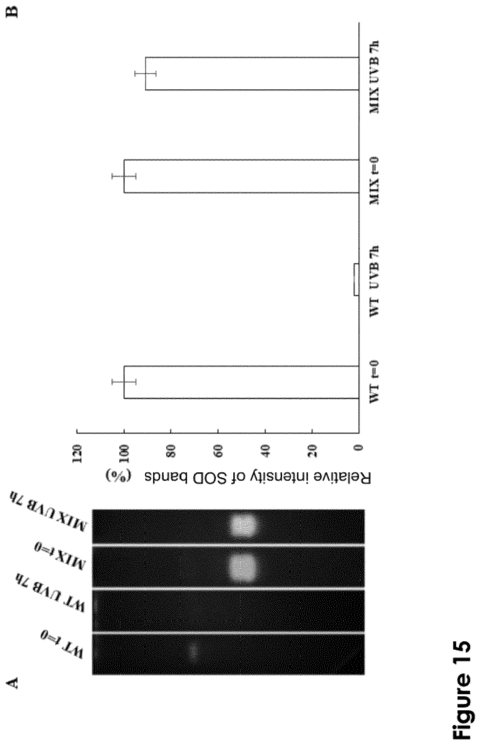

**Figure 15** shows in: (A) an image of a polyacrylamide gel after electrophoresis in non-denaturing conditions (native-PAGE) that shows the effect of the exposure to UVB rays for 7 hours on protein extracts from untransformed tomato cells (WT) and on the mixture (MIX) consisting of the extracts from tomato cells containing SsSOD, ApSOD and DrSOD. The bands of SOD activity were revealed by staining; (B) a histogram graph relating to the densitometric analysis of the intensity of the SOD activity bands of the gel shown in A (the signal intensity of the SOD activity band after 7 hours of UVB treatment was reported as percentage value with respect to the intensity of the signal of each SOD activity band at time=0, corresponding to 100%).

### Detailed description

The present invention describes a composition comprising plant cell culture extracts containing superoxide dismutase of extremophile bacteria. The extremophilic bacteria proliferate in extreme physical and/or geochemical conditions, which are deadly for most of the living organisms on earth.

The extremophilic bacteria are classified into categories including: thermophilic bacteria, able to survive in high temperature environments; acidophilic bacteria, able to survive in acidic environments; halophilic bacteria, able to survive in environments with high salt concentrations; alkalophilic bacteria, able to survive in basic environments; psychrophilic bacteria, able to survive in low temperature environments. Typical habitats colonized by these particular bacteria include thermal springs, underwater hydrothermal systems, salt lakes and salines, cold seas, polar soils and alpine glaciers, as well as the deep-water sediments.

These extremophilic microorganisms contain enzymes, so-called "estremozymes", which are able to maintain their structure and catalytic activities in the aforementioned extreme environmental conditions; among said estremozymes, there are also superoxide dismutases.

In order to exploit the stability characteristics of this class of enzymes, the Applicants isolated superoxide dismutase (SOD) from three different extremophilic microorganisms: *Sulfolobus solfataricus*, *Aeropyrum pemix* and *Deinococcus radiodurans.*

*Sulfolobus solfataricus* is a thermo-acidophilic bacterium, which lives in extremely acid (pH between 2 and 4) and hot (80°C) conditions, like the geysers.

*Aeropyrum pemix* is a thermo-halophilic bacterium, isolated from the marine sediments off the coasts of Japan, it grows at temperatures between 70 and 100°C and at a salinity between 1.8 and 7% (optimum, 3.5%).

*Deinococcus radiodurans* is one of the most radioresistant organisms known in the world. It is able to withstand very high doses of radiation that are incompatible with the life of any other organism. Moreover, it is able to survive in extreme cold, dehydration and acidic conditions and, for this reason, it is also considered a poly-extremophilic bacterium.

Superoxide dismutase genes of the aforementioned extremophilic microorganisms have been isolated and expressed first in the bacterial system model *E.coli*, in order to study their properties, and then expressed in plant cell cultures, in order to produce plant extracts containing the superoxide dismutase estremozymes.

In particular, cell cultures represent a very advantageous option for the expression of heterologous proteins. In fact, the extracts obtained from plant cell cultures are rich in secondary metabolites, of particular interest for the production of active ingredients for cosmetic applications (Barbulova et al., 2014).

In one embodiment, the plant cultures in which the extremophile superoxide dismutases are expressed is tomato, *Solanum lycopersicum,* due to the previously described beneficial properties. Alternatively, it is possible to use cultures derived from cells of other plant species (for example, *Nicotiana*, *Rubus*, *Coffea*, *Vitis*, *Lotus* cells) to produce extremophile SOD: several plant cultures have already been used successfully to produce extracts rich in beneficial substances and have got proven efficacy for cosmetic/dermatological applications (Apone et al., 2017).

The composition of the present invention shows a stable antioxidant activity thanks to the presence of the superoxide dismutases isolated from the aforementioned microorganisms.

In fact, it shows enzymatic activity even at high temperatures (up to 90°C), at low pH (up to 1.0), at a high concentration of salts (up to 7%) and at high doses of UVC radiation (λ=254 nm and E=10 J/cm² up to 7 hours of exposure) and UVB (λ=300-315 nm, E=0.6 mW/cm2 up to 7 hours of exposure).

Thanks to this property, the present composition is suitable for the use in industrial applications where extreme physical factors can be decisive for the structural stability of the proteins, including enzymes. Indeed, the maintenance of the protein structure is essential so that a protein, such as superoxide dismutase, is able to exercise its biological activity.

Furthermore, these enzymes obtained from extremophilic microorganisms are expressed and contained in extracts of tomato cell cultures, that are totally free of allergenic or irritating substances (such as, for example, the alkaloids present in plants leaves and fruits ) and, therefore, do not present any potential risk of allergic reactions in humans, neither for application on the skin (and other tissues) nor for ingestion.

In particular, the most interesting application sectors are represented by dermocosmetic, nutraceutical and food preservation sectors.

In dermocosmetics, the present composition can be particularly useful in soothing or skin protection products suitable to contrast oxidizing agents, thanks to the presence of the SODs. The use of the composition directly in sunscreens may be of greater interest, thanks to heat and UV rays stability of extremophile SODs. In fact, this would guarantee an antioxidant action even during prolonged exposure to sunlight, conditions in which the stability of mesophilic enzymes or anti-oxidant compounds commonly used in cosmetic products may be compromised. In the nutraceutical sector, the present composition is particularly interesting because it is able to exert its antioxidant action even at low pH values (from 1 to 3), which are typical of stomach gastric fluids. In fact, numerous oxidizing agents contained in foods exert an inflammatory action on the tissues of stomach and intestines, often resulting in more serious pathological conditions. Furthermore, natural substances or enzymes with antioxidant action are hydrolysed or inactivated by acids produced by the gastric wall. Advantageously, the extremophilic enzymes of the present composition show a high resistance to acid pH and proteolytic action of gastric enzymes, and this allows their use in nutraceutical products or food supplements for human or animal use.

In the canning sector, the extracts contained in the composition of the present invention show a stable antioxidant activity even in conditions of high salinity and high temperature, typical of the food preservation processes, thanks to the presence of *Sulfolobus solfataricus* (SsSOD) or *Aeropyrum pemix* (ApSOD)superoxide dismutase . The food products treated with the composition of the present invention maintain their organoleptic properties unaltered, as they are protected by oxidation phenomena.

The composition of the invention also includes vehicles, solvents, excipients and/or adjuvants. Furthermore, it can be in the form of gels, creams, lotions for cutaneous application, or in the form of pills, tablets, powders, solutions for biomedical/nutraceutical applications, and, finally, in the form of aqueous solutions for application in the food field, in particular for food preservation.

By way of non-limiting examples of the present invention, below there are examples relating to the preparation of the plant cell culture extracts of the species *S*. *lycopersicum* (tomato), which express and contain superoxide dismutases isolated from extremophilic microorganisms, and their biological activities aimed to suppress the damage caused by the action of oxidizing agents.

### EXAMPLES

### Example 1.

*Expression in tomato cell cultures of the superoxide dismutase (SOD) belonging to the microorganism Sulfolobus solfataricus and characterization of the cell-derived extract.*

For the expression in tomato cells (MicroTom variety) of the gene encoding for superoxide dismutase (SOD) of the extremophilic microorganism *Sulfolobus solfataricus* (SsSOD) **(SEQ ID NO 1),** the binary vector pBI121 engineered with the aforementioned gene was transferred into cells of *Agrobacterium tumefaciens* (LBA4404 trunk) for the genetic transformation of tomato cotyledons (*S*. *lycopersicum*)*.*

The infected plant tissues were then transferred onto a specific culture medium for the induction of calluses, said medium containing the antibiotic kanamycin for selecting transformed cells. After about 6 weeks from the genetic transformation, a large number of calluses were regenerated, isolated from the remaining plant tissue, transferred onto fresh medium and analyzed by semi-quantitative RT-PCR. **Fig.1A** shows the analysis related to some SsSOD expressing lines . By using specific oligonucleotides for SsSOD sequence, te obtained amplification products were normalized to amplified 18S ribosomal RNA obtained in the same reaction (multiplex PCR). All the analyzed clones (1-5) show a high level of SOD expression while, as expected, no amplification band was found in the untransformed tomato cells (WT) used as a control. The cellular lines were analyzed for the presence of the protein by Western Blot (WB) with a specific anti-SOD antibody. The result of the Western Blot is reported **in** **Fig. 1B****:** in the analyzed lines, the presence of the SsSOD protein of the expected molecular weight (24kDa) was highlighted, in particular in line 1, while the protein was absent in the control cells (WT).

Line 1 cells were grown in liquid culture, collected and processed to obtain an extract containing SsSOD. The extract was characterized for its antioxidant activity, which is mainly due to the presence of the recombinant enzyme. An electrophoretic analysis of the extract of wild-type cells (WT, not-expressing SsSOD) and line 1 expressing SsSOD (SsSOD#1) was performed under native conditions (Native-PAGE) and subsequent activity staining **(****Figure 2****).** The analysis on the native gel shows that, a band corresponding to an endogenous SOD, having a molecular weight similar to SsSOD, is present also in the WT sample; however, it is much less intense than that present in the SsSOD#1 sample, indicating that in the latter the antioxidant activity was much stronger.

Through the optimization of the assay conditions for the enzymatic activity dosage, the recombinant SsSOD present in the extract of line 1 was characterized, determining the main functional parameters, in terms of activity, by varying pH and temperature values. As shown in **Figure 3****,** it was possible to determine the optimal conditions for the activity of the extracts, which turned out to be a pH value equal to 5 and a temperature of 70°C (value measured with a spectrophotometric activity assay). Furthermore, the enzymatic activity of the extracts containing SsSOD was significantly higher than that of WT extracts, both in the temperature range from 57 to 90°C and in the pH range from 1 to 8, indicating that SsSOD guarantees that the extracts of the tomato cells preserve an antioxidant activity under extreme temperatures and pH conditions, which, instead, wild-type extracts do not show.

### Methods

### a) Cloning of Sulfolobus solfataricus superoxide dismutase genes.

### - Genomic DNA purification.

For the isolation of DNA from archaeobacteria *Sulfolobus solfataricus*, an adaptation of a devotedprotocol was used (Sulfolobus protocols, Stedman Lab, http://web.pdx.edu) as summarized below:
a) pellet obtained from 1 ml of culture in the stationary growth phase was placed in a 2 ml tube and resuspended in 250 µL of TEN (10 mM Tris/HCl pH 8.0, 1 mM EDTA, 150 mM NaCl);
b) 250 µl of TENST (TEN added with 0.12% Triton X-100 and 1.6% Sarcosine) were added and the mixing was repeated by inverting the tube;
c) after incubation for 30 minutes at room temperature, 500 µl of the ternary phenol/chloroform/isoamyl alcohol mixture (25:24:1) were added and the mixture was vortexed for 2 minutes at the maximum strength (40 hertz);
d) a heavier organic phase (bottom), a solid phase (intermediate) and an aqueous phase (supernatant) were separated, and 50 µl of 3M sodium acetate and 1 ml of ethanol were added to the latter;
e) after an incubation for 15 minutes on ice, the tube was centrifuged (speed 14000 rpm) for 15 minutes and a precipitate consisting of genomic DNA and RNA was obtained; then, the supernatant was removed and the precipitate was washed with 500 µl of 70% ethanol;
f) the precipitate was allowed to air dry for 15 minutes and, subsequently, dissolved in a 15 µl TE buffer solution containing ribonuclease A (RNAsi A) enzyme; after 30 minutes incubation at 37°C, the DNA preparation was checked by electrophoresis on 1% agarose and the concentration was measured by a fluorometer. Approximately 5 µg of genomic DNA of the microorganism of interest were obtained.

### - Primer design.

*Sulfolobus solfataricus* genome, as well as *Aeropyrum pemix* and *Deinococcus radiodurans* ones, has been completely sequenced and available in databases, so primers for the PCR amplification of the selected genes can easily be designed.

Primers must allow the amplification of the entire coding regions; therefore, the "forward" primer has to start from the ATG codon of the initial translation, while the "reverse" primer has to start from the stop codon of the same coding sequence. Furthermore, in order to make the amplification conditions sufficiently stringent, primers have been designed with an *annealing* presumed temperature in the range of 52-60°C.

The primers for the amplification of *Sulfolobus solfataricus* superoxide dismutase are the following:

| Primer | Sequence | Temperature annealing (°C) | Gene |
|---|---|---|---|
| Forward | SEQ ID No 4 | 54.9 | sod (locus SSO_0316) |
| Reverse | SEQ ID No 5 | 52.6 | sod (locus SSO_0316) |

### - Amplification reactions.

The PCR reaction for the amplification of superoxide dismutase gene was carried out using the high-fidelity Pfu DNA polymerase "Phusion Green Hot Start II High-Fidelity" (Thermo Fisher Scientific), the primers of the sequence SEQ ID No 4 and SEQ ID No 5 and the microbial DNA as a template.

Using the recommended conditions for "GC-rich" substrates, 20 µl of reaction mixtures containing 25-50 ng of DNA, 200 µM for each dNTP, 0.5 µM for each *primer*, DMSO at 3%, and 0.4 units of the DNA polymerase enzyme were prepared and subjected to the following amplification program: an initial denaturation cycle at 98°C for 30 seconds, subsequent 30 cycles consisting of a denaturation phase at 98°C for 10 seconds, an "annealing" phase at a temperature between 54 and 60°C for 30 seconds, an extension phase at 72°C for 45 seconds, and a final extension cycle at 72°C for 7 minutes.

### - Purification of the amplified DNA and sequencing.

The amplified DNA was purified using the system and the procedure of "Zymoclean Gel DNA Recovery" product (Zymo Research), which allow to isolate the DNA starting from agarose gel fragments containing the amplicons of interest, by means of affinity chromatography columns. Aliquots of these DNA were used for the sequence analysis, obtained as a service by a specialized laboratory, which confirmed the identity with the genes of interest.

### - Cloning of genes in plasmid vectors and transformation of agrobacterium.

The purified DNA was cloned in a plasmid vector for its preservation and propagation, by "StrataClone Ultra Blunt PCR Cloning Kit", a specific kit for DNA amplified with Pfu DNA polymerase, with high yields guaranteed by the technology exploiting DNA topoisomerase I properties.

Then, plasmids containing the SOD gene were verified by means of enzymatic restriction analysis.

The cloned gene was transferred into plant expression vectors by "SureVector Cloning System" (Agilent), which was based on the assembly of the construct in a single reaction containing the gene of interest and synthetic modules corresponding to the various components of the vector (promoter, selection marker, origin of replication, possible N- and/or C-Tag), which were combined into the correct sequence by recognizing between the terminal regions of each fragment.

The gene of interest was prepared for the assembly reaction by amplification with chimeric primers, which contained terminal portions necessary for the correct placement in the final construct. Following this procedure, the expression vector Sure-SsSOD was prepared, containing the T7 promoter, a gene for ampicillin resistance, and the origin of replication with a high number of pUC copies. The vector was inserted into the bacterium *Agrobacterium tumefaciens* by genetic transformation.

For the transformation of the agrobacterium (LBA4404 trunk), made competent with CaCl₂, the freezing-thawing technique was used. To aliquots of competent cells (about 100 µl), 1 µg of DNA of vector Sure SsSOD was added; the cells were first placed in ice for 5 minutes, then at the temperature of -130°C for 5 minutes and finally at 37°C for 10 minutes. 800 µl of liquid YEP (Yeast extact 10g/l, peptone 10g/l, NaCl 5g/l, pH 7.2) was added to the cells and incubated at 28°C for 4 hours. The cultures were then plated on solid YEP, supplemented with the antibiotic spectinomycin (100mg/l) for the selection of recombinant agrobacterium cells.

### b) Transformation of tomato cotyledons with the SOD genes and callus induction.

Tomato cotyledons (*MicroTom* cultivar), 10-12 days old, were left to germinate on medium MS30 (Murashige and Skoog Basal Medium, 4.4 g/L, sucrose 30 g/L, pH 7.2) after being sterilized in 70% ethanol (1 minute), 1% sodium hypochlorite (15 minutes) and then rinsed 3 times in sterile distilled water. The bacterium *Agrobacterium tumefaciens* (LBA4404 trunk) was grown for 18 hours at 28°C in 10 ml of liquid YEP, then centrifuged at 4000 rpm for 5 minutes at room temperature and the pellet was resuspended in 10 ml of AM1 medium based on B5 Gamborg medium (micro and macro-elements including vitamins, Duchefa, Netherlands) containing: myoinositol 500 mg/L, hydrolyzed casein 500 mg/L, sucrose 30 mg/L, 2,4D 1 mg/L, kinetine 0.1 mg/L, NAA (naphthyl acetic acid) 0.01 mg/L, adenine 1 mg/L, pH 5.7.

The co-cultivation of cotyledons with agrobacterium took place in the Petri dish for 15 minutes with the addition of acetosyringone (370 µM). The explants were transferred to the solid AM 1 medium and placed in a growth chamber (24°C with a photoperiod of 16 hours of light and 8 hours of darkness) for two days. Then, these were transferred to liquid AM1 with the added antibiotic cefotaxime (500 mg/l) for 30 minutes to remove the agrobacterium from the explants. The latter were further washed with the medium AM1 alone and then transferred to Petri dishes containing AM1 with 250mg/l cefotaxime and kanamycin (50 mg/l) for the induction and selection of the transformed cells. After about 4 weeks from the transformation, the first calluses appeared around the explants; once the size of 0.5 cm was reached, the calluses were excised from the leaf tissue and transferred to fresh AM1 containing the cefotaxime and kanamycin antibiotics.

### c) Characterization of the transformed tomato cell lines.

The transformed tomato calluses were analyzed by semi-quantitative RT-PCR in order to evaluate the expression levels of the transgene.

For this purpose, 0.5 ml of cells were centrifuged to remove culture medium and the cellular pellet was used to extract total RNA using an extraction kit "RNeasy Plant Mini Kit" (Qiagen). The RNA samples were subjected to a treatment with DNasi I (Ambion) in order to eliminate the contaminating genomic DNA. 2 µl of each sample was loaded on 1% agarose gel in the presence of "loading dye" (0.25% bromophenol Blue, 0.25% xylene cyanol FF, 40% sucrose in water) and quantified using a specific quantitative marker for RNA as reference (Riboruler RNA ladder, Fermentas). The Gene tools software (Perkin Elmer) was used for the quantification. 300 ng of total RNA were reverse transcribed using Transcriptase Reverse enzyme (Thermo-Scientific).

The semi-quantitative RT-PCR reactions were conducted using as internal standards the primer pair "universal 18S primer/competimer" (Ambion) in a 3:7 ratio.

The amplification reaction was conducted in a volume of 25µl with 0.4 µM forward primer (Fw), 0.4 µM reverse primer (Rev) and 1.25 units of Wonder Taq polymerase enzyme. The amplification cycles used consisted of an initial denaturation at 95°C for 1 minute, followed by 35 cycles of: denaturation at 95°C for 15 seconds, annealing at 50°C for 15 seconds and extension at 72°C for 30 seconds.

The PCR products were separated on 1.5% agarose gel, visualized by Geliance tool (Perkin Elmer) and densitometrically analyzed by Genetools software. The values shown in the graph of Figure 10 represent ratios between the intensity of the band relative to the analyzed gene and that of the band relative to the standard reference (18S ribosomal RNA).

The primer sequences specific for the amplification are SEQ ID No 10 (Fw primer) and SEQ ID No 11 (Rv primer).

To verify the presence of the recombinant protein, the cells were collected and total protein extracted, both from wild type and transgenic cells, with 300 µl of buffer containing Tris-HCl 0.125 M pH 6 and 4% SDS with the addition of a mix of plant protease inhibitors (Sigma). After mechanical grinding of calluses in the buffer, the cells were centrifuged at 14000 rpm for 10 minutes at 4°C. The supernatant was then transferred to new tubes (Eppendorf) and the spectrophotometer quantification of the extracted proteins was carried out with Bradford reagent.

20 µg of protein was separated on 18 % SDS-PAGE and a Western Blot was conducted on a PVDF membrane (Millipore) with the specific anti-SOD antibody diluted 1:1000 in 1X TBS with 5% Blotting-Grade Blocker (Bio-Rad). The secondary antibody (anti-Rabbit), conjugated to horseradish peroxidase (HRP), was used at a dilution of 1:5000 and the blot was developed with the "Pierce ^{™} 1-Step Ultra TMB blotting-solution" reagent (Bio-Rad). As a positive control of the hybridization with the antibody, an extract of *E*. *coli* expressing *D. radiodurans* SOD was used (indicated as C+ in the Western Blot analysis).

### d) Preparation, growth and collection of liquid cultures

Upon reaching about 2 cm growth in solid medium(weighing about 50 mg), the calluses selected on exogenous SOD expression levels were taken and placed in flasks containing 150 ml of liquid AM1 medium with antibiotics.

The flasks were placed in a growth chamber at a temperature varying between 24 and 26°C on an orbital rotation shaker of 120 rpm. After about 4 weeks, the desired density was reached, namely about 300 g/L, which allowed cell collection for the subsequent processing. The cell collection was performed through filters with controlled porosity, in order to remove the culture medium. The cells were subsequently washed in distilled sterile water and frozen at -80°C. The extracts of *S*. *lycopersicum* cells were prepared by the following procedure.

*S*. *lycopersicum* cells (100 g) were suspended in a cold saline buffer PBS 1X (NaCl 136 mM, KCl 2.7 mM, NaH₂PO₄ 12 mM, KH₂PO₄ 1.76 mM, pH 7.4) in a 4:1 volume/weight ratio (approximately 400 ml of buffer per 100 g of fresh cells) and homogenized in a mechanical blender for 15 minutes. The term "homogenization" means a treatment of grinding of the plant material, which takes place in a suitable container, such as a ceramic mortar and a ceramic pestle, previously cooled, or for larger volumes, larger containers may be used, even metal, where the material can be homogenized with metallic blades, using blenders or laboratory or industrial presses.

After obtaining a homogeneous lysate, the suspension was centrifuged at 4000 g for about 15 minutes at 4°C and filtered to remove further residues and recover the supernatant (about 700 mL).

The supernatant was dried by a freeze-drying process until the solvent was completely removed, obtaining about 3 grams of powder. As an alternative to freeze-drying, drying chamber or spray dryer may be used.

### e) Analysis of SOD enzymatic activity in tomato cell extracts.

For the dosage of SOD enzymatic activity in the plant extracts, a spectrophotometric assay and an assay on acrylamide gel in non-denaturing conditions (Native-PAGE) were carried out followed by activity staining.

### - The spectrophotometric test

The antioxidant activity of SOD-expressing extracts was tested for their ability to inhibit the photochemical reduction of colorless Nitro blue Tetrazolium (NBT) to violet coloured formazan.

The reaction mixtures (final volume of 260 µl) containing 2 µg of plant extract obtained from untransformed tomato cells (WT), or from those transformed with the genes encoding the various extremophilic SODs, were pre-incubated for 20 minutes at 37°C with H₂O₂ (20 µM), which acted as an inhibitor of the Cu/Zn- and Fe-SOD isoforms in Tris-HCl 50 mM pH 8.0. After the pre-incubation, the following was added to the reaction mixture: EDTA (0.1 M) and NBT (1.5 mM) for a final volume of 1 ml. The cuvette containing this solution was exposed to light for 8 minutes, then riboflavin (0.12 mM) was added and the mixture was exposed to light for the next 12 minutes to allow the development of the photochemical reaction. A color change of the samples to purple represented a clear sign of the oxidation of the NBT to formazan. The activity of the extracts containing the extremophilic superoxide dismutase was determined by measuring their ability to inhibit the photochemical reduction. The violet color developed in the reaction was measured spectrophotometrically at 560 nm. In particular, the SODs contained in the extracts subtract superoxide anion, thus inhibiting the reduction of NBT and determining a reduction of the absorbance at 560 nm. The reduction rate of NBT was linear in the first minutes of reaction. The activity dosage was carried out in triplicate on different enzymatic samples.

The protein content in the plant extracts was determined according to the Bradford method (Noble, 2014), using bovine serum albumin (BSA) as a standard.

The spectrophotometric assay was used to carry out the biochemical characterization of the transformed and untransformed plant protein extracts under different temperatures, pH and salt concentrations.

Following the treatment with H₂O₂, the reaction mixtures (final volume 260 µL) were pre-incubated for 15 minutes under different temperature conditions (70, 80 and 90°C), pH (pH = 3.0 with 50 mM glycine-HCl; pH = 5.0 with 50 mM sodium acetate; pH = 8.0 with 50 mM Tris-HCl) or salt concentration (0.5, 1, 1.5 M NaCl). The pre-incubation temperature used for the experiments at different pH and salt concentration conditions was 37°C. After the pre-incubation, the NBT/Riboflavin assay was conducted as already described above.

### - SOD activity test on acrylamide gel in non-denaturing conditions (Native-PAGE).

SOD antioxidant activity was determined on 10% polyacrylamide gel by a specific staining, following the electrophoretic run performed in non-denaturing conditions (in the absence of SDS, beta-mercaptoethanol and boiling). Under these conditions, proteins retain their native structure and enzymes retain their activity.

At the end of the electrophoretic run, the gel was immersed in a solution containing Nitro blue Tetrazolium (NBT, 306 µM) and Riboflavin (265 µM) and incubated at room temperature for 15 minutes in the dark (Beauchamp and Fridovich, 1971). The reaction was then stopped by adding 0.1% TEMED and incubating the gel for another 15 minutes in the dark. SOD activity was revealed through the appearance of light bands on the gel, deriving from the missing reduction of NBT due to the dismutation of O₂ anion by the enzyme. The same amount of total protein was loaded on each lane (indicated in the legends of the figures), estimated by the Bradford method.

To study the resistance of tomato cell extracts to UVB rays, reaction mixtures (final volume of 50 µl) containing 10 µg of the plant protein extracts in Tris-HCl 50 mM pH 8.0 were exposed to UVB rays (300-315 nm) at a distance of about 4 cm from the light source and irradiated up to 7 hours in ice to avoid evaporation of the samples during the irradiation. At the end of the exposure, the reaction mixtures were loaded on the gel and analyzed as described above.

### Example 2

*Expression in plant cell cultures of SOD belonging to the micro-organism Aeropyrum pemix and characterization of the cell-derived extract.*

The genetic transformation of tomato with *Aeropyrum pemix* SOD (ApSOD) (SEQ ID NO 2) was performed as described in Example 1, using *Agrobacterium tumefanciens.* Four transformed lines were analyzed by RT-PCR using specific primers for the ApSOD and comparing the amplification products with the amplified 18S ribosomal RNA obtained in the same reaction (multiplex PCR) (**Figure 4A**). All the analyzed clones showed a high level of expression of the SOD while, as expected, no amplification band was found in the unprocessed tomato cells (wild type, WT) used as control.

From the Western Blot analysis, it was shown that the transgenic lines contained detectable amounts of the ApSOD protein, while in the untransformed line a slight hybridization band was barely visible due to the high similarity between the endogenous tomato SODs and the extremophilic SOD (**Figure 4B**).

From line 2 expressing ApSOD, a water-soluble extract was produced and used for the characterization of the antioxidant and biochemical activity of extremophilic SOD. The electrophoretic analysis in native conditions (native-PAGE) and the subsequent activity staining showed the strong activity of the recombinant SOD present in line 2 compared to WT sample (**Figure 5**). Subsequently, a study of the enzymatic activity of the tomato cell extract containing ApSOD was carried out, varying the temperature and salinity conditions (spectrophotometric analysis).

The results shown in **Figure 6A and Figure 6B** showed that the optimal conditions of the enzymatic activity occured at a temperature of 80°C and a salt concentration of 1M, factors that denoted the hyperthermophilic and halophilic nature of the recombinant enzyme; under the same conditions, the WT extracts showed a very low enzymatic activity.

### Methods

### a) Cloning of Aeropyrum pemix superoxide dismutase genes.

### - Purification of genomic DNA

The purification of *Aeropyrum pemix* genomic DNA was performed as described in Example 1.

### - Primer design

The primers for the amplification of the superoxide dismutase of *Aeropyrum pemix* were the following:

| Primer | Sequence | Temperature annealing (°C) | Gene |
|---|---|---|---|
| Forward | SEQ ID No 6 | 56.3 | sod (locus APE_0741) |
| Reverse | SEQ ID No 7 | 57.1 | sod (locus APE_0741) |

### - Amplification reactions.

The amplification reaction was performed according to the method of Example 1, using the primers of sequences SEQ ID No 6 and SEQ ID No 7.

### - Purification of the amplified DNA, sequencing and cloning of genes in plasmid vectors and transformation of Agrobacterium.

The purification of the amplified DNA and its cloning in plasmid vectors used for the transformation of *Agrobacterium tumefaciens* were performed according to the method of Example 1.

### b) Transformation of tomato cotyledons with SOD genes and callus induction.

The transformation of tomato cotyledons was carried out according to the method of Example 1.

### c) Characterization of the processed tomato cellular lines.

The characterization of the transformed cellular lines was carried out according to the method of Example 1, using the primers having sequence SEQ ID No 12 (Fw primer) and SEQ ID No 13 (Rv primer).

### d) Preparation, growth and collection of liquid cultures

The preparation, growth and collection of liquid cultures were carried out according to the method of Example 1.

### e) Analysis of SOD enzymatic activity in tomato cell extracts.

The analysis of superoxide dismutase enzymatic activity of the tomato cell culture extracts was carried out according to the method of Example 1.

### Example 3.

*Expression in plant cell cultures of SOD belonging to Deinococcus radiodurans microorganism and characterization of the cell-derived extract.*

*Deinococcus radiodurans* SOD (DrSOD) (SEQ ID NO 3) was stably transferred to tomato via *Agrobacterium tumefaciens.*

The calluses selected on selective medium were analyzed by RT-PCR using specific primers for the SOD transferred sequence. **Figure 7A** shows the RT-PCR of 5 transgenic lines. The amplification products obtained using specific oligonucleotides for DrSOD sequence were normalized to the amplified 18S ribosomal RNA obtained in the same reaction (multiplex PCR). All the analyzed clones showed a high level of SOD expression . The lines were then analyzed in Western-blot and, as shown in **Figure 7B****,** the transgenic tomato lines expressed DrSOD at high levels, whereas in the WT cell extract a slight band corresponding to an endogenous SOD is present.

The extract of line 3 containing DrSOD was used for the characterization of antioxidant and biochemical activity of the extremophilic SOD. The Native-PAGE analysis followed by activity staining of the samples confirmed the highest antioxidant activity of the extract of line 3 compared to WT (**Figure 8**). By the figure it was also evident that the SOD present in DrSOD#3 line migrated faster on the gel than the endogenous one present in the WT line, and, moreover, it appeared as a double band, probably because a part of the recombinant protein was as glycosylated form, thus having a different molecular weight than the unmodified one.

The study of the antioxidant activity of line 3 cell extract containing the recombinant DrSOD was carried out by determining the main biochemical parameters in terms of resistance to UVB radiation. The results shown in **Figure 9** **(A and B)** showed that the extract containing DrSOD showed an exceptional resistance to UVB rays after 7 hours of exposure (preserves 90% of activity) compared to WT (preserves only 20% of activity ), a behavior that denoted the extremophilic nature of the present recombinant enzyme.

### Methods

### a) Cloning of Deinococcus radiodurans superoxide dismutase genes.

### - Purification of genomic DNA

The purification of *Deinococcus radiodurans* genomic DNA was performed according to the system and the method of "PureLink^{®} Genomic DNA Mini Kit" (Invitrogen), which involved the use of chromatographic columns containing a hydrophobic resin that retained the DNA by affinity. Starting from about 5 x 10⁸ cells obtained from an overnight growth, a quantity of genomic DNA equal to 1 µg was obtained.

### - Primer design

The primers for the amplification of the superoxide dismutase of *Deinococcus radiodurans* were the following :

| Primer | Sequence | Temperature annealing (°C) | Gene |
|---|---|---|---|
| Forward | SEQ ID No 8 | 58.1 | sodA (locus DR_1279) |
| Reverse | SEQ ID No 9 | 59.7 | sodA (locus DR_1279) |

### - Amplification reactions.

The amplification reaction was performed according to the method of Example 1, using the primers of sequences SEQ ID No 8 and SEQ ID No 9.

### - Purification of the amplified DNA, sequencing and cloning of genes in plasmid vectors and Agrobacterium transformation.

The purification of the amplified DNA and its cloning in plasmid vectors used for the transformation of *Agrobacterium tumefaciens* were performed according to the method of Example 1.

### b) Transformation of tomato cotyledons with SOD genes and callus induction.

The transformation of tomato cotyledons was carried out according to the method of Example 1.

### c) Characterization of the transformed tomato cell lines.

The characterization of the transformed cell lines was carried out according to the method of Example 1, using the primers having sequence SEQ ID No 14 (direct primer) and SEQ ID No 15 (reverse primer).

### d) Preparation, growth and collection of liquid cultures

The preparation, growth and collection of liquid cultures were carried out according to the method of Example 1.

### e) Analysis of the enzymatic activity of the SODs in the tomato cell extracts.

The analysis of superoxide dismutase enzymatic activity of the tomato cell culture extracts was carried out according to the method of Example 1.

### EXAMPLE 4 (not according to the invention)

*Analysis of antioxidant activity of the plant extracts including extremophile bacteria SOD on human fibroblasts.*

The ability of the tomato extracts containing extremophile SOD to protect human cells from the adverse effects of oxidative stress, one of the main causes of cellular senescence, was tested.

For this purpose, human fibroblasts were subjected to oxidative stress for two days in the presence of H₂O₂ and treated with the WT tomato cell extracts and the transgenic extracts SsSOD#1, ApSOD#2, DrSOD#3. The expression level of the senescence marker p21 gene was analyzed by RT-PCR analysis.

The specific PCR products were normalized to the internal standard (18S ribosomal RNA ) and the average values obtained from three independent experiments were converted into percentage, assigning to the treatment with H₂O₂ a value equal to 100% (**Figure 10**).

The expression analysis of p21 indicated that cells treated with SOD-expressing extracts were less senescent than cells treated only with H₂O₂ or those added with the WT cell extract; in fact, the expression of p21 was significantly reduced by 31%, 36% and 15% in the cells treated with extracts expressing SsSOD, ApSOD and DrSOD, respectively.

Therefore, the treatment of the cells with the extracts containing extremophile SOD significantly reduced the stress caused by H₂O₂, determining a slowing of cellular senescence. These results, therefore, indicated that extracts containing the extremophile SOD, in particular those containing SsSOD and ApSOD, due to their characteristics of greater stability, could be potentially used in applications related to human well-being (nutraceutical and biomedical sector) to oppose the damages of the oxidative stress on cells.

### Methods

*Molecular and cellular assays on human fibroblasts.*

For the induction of senescence and expression analysis in human fibroblasts (HDF), the cells were seeded in 6-well plates at a density of 8 x 10⁴, treated with WT and transgenic tomato cell culture extracts (at a concentration of 0.02%) and subjected to stress for two days with H₂O₂ (100 µM), twice a day for 1 hour.

RNA was extracted from the cells using the "GenEluteTM Mammalian Total RNA Miniprep kit" (SIGMA) and processed as described in Example 1.

The amplification reaction (PCR) was conducted in a volume of 25 µl composed of 1X "Wonder Taq Reaction buffer" containing 1mM dNTPs (deoxynucleoside triphosphates), 0.4 µM direct primer (forward, Fw), 0.4 µM reverse primer (reverse, Rev), 1.25 units of the enzyme "Wonder Taq". The amplification cycle was composed of an initial denaturation at 95°C for 1 minute, followed by 35 denaturation cycles at 95°C for 15 seconds, annealing at 53°C for 15 seconds and extension at 72°C for 30 seconds.

The sequences of the primers specific for the amplification of the Cdkn1A gene (p21) are shown below:
Cdkn1A direct primer (Fw): SEQ ID No 16
Cdkn1A reverse primer (Rv): SEQ ID No 17

The PCR products obtained were analyzed as previously reported. The average values obtained from three independent experiments were converted into percentages, assigning to the treatment with H₂O₂ a value equal to 100%.

### EXAMPLE 5 (not according to the invention)

*Study of the effect of tomato cell extract expressing DrSOD on the protection of nuclear DNA in human epidermis keratinocytes.*

UV radiations are among the factors that cause most of the damages to skin cells, due to oxidative stress.

For a cosmetic/dermatological application of the composition of the present invention, the antioxidant activity of the extract containing DrSOD was measured under extreme conditions of UV rays and heat.

In particular, it has been verified if the treatment with the tomato cell culture extract containing DrSOD, even after prolonged exposure to UV, preserved its protective effect on nuclear DNA against the damage caused by H₂O₂ in human keratinocytes. This was evaluated by the Comet assay (it is specific for the measure of the fragmentation level of the genomic DNA by direct observation of the cells under an optical microscope).

To obtain quantitative data on the protective activity of this extract, the length of 50 "tails" (comet tail) of the cell nuclei, index of DNA fragmentation, was measured and an average value was calculated. As shown in the graph of **Figure 11****,** the average length of the "tail" of the cellular nuclei treated only with H₂O₂ was greater than the untreated control cells. The cells treated with WT extracts showed a reduction of nuclei fragmentation, indicating that the extract of WT cells already exerted a partial protective effect on the keratinocytes (reduction of the tail of about 23%); however, this effect was abolished when the same extracts were pre-treated with the UVs for 7 hours.

In contrast, the transgenic DrSOD extracts, derived from line 3, not only had a greater protective effect on the cells subjected to stress with H₂O₂, (reduction of the comet tails by about 37%), but after the treatment of the extract with UV for 7 hours, they remained active, guaranteeing a significant reduction of the nuclear degradation (reduction of the comet tails of 28.5%). These results, therefore, indicated that the extract containing DrSOD, due to its characteristics of high resistance to UV rays, could be potentially used in sunscreen products for the skin treatment against oxidative stress.

### Methods

### "Comet" assay on human keratinocytes

1.5 x 10⁵ HaCaT cells per well were grown in 6-well plates in DMEM culture medium (Lonza), supplemented with 10% fetal bovine serum (FBS) for 24 hours. The keratinocytes were first treated with tomato extracts expressing extremophile SODs and the control extracts overnight, then they were subjected to stress with H₂O₂ 300 µM for 3 hours.

Afterwards, the cells were removed from the plate using 1 ml/well of "Cell Dissociation Solution Non-enzymatic" (Sigma) and transferred into 1.5 ml tubes (Eppendorf) to be centrifuged at 1500 rpm for 5 minutes.

The cells were washed in PBS 1X and resuspended in about 10-20 µl PBS, depending on the amount of cells recovered. 80 µl "Low Melting point Agarose" (LPMA) 0.5% in PBS was added to the cells, balanced at 37°C. The dispersed cells were placed on a slide previously covered with a thin layer of "Normal Melting Agarose" (NMA). A cover slip was placed over a drop of cells, and the slides were then placed in the fridge at 4°C until the layer of agarose with the cells solidified completely (10-15 minutes). The cover slips were removed from the agarose, and the slides placed in a cold lysis solution (2.5 M NaCl, 100 mM EDTA, 10 mM Trizma Base pH 8.5, 1% TritonX-100) and left for about 2 hours at 4°C.

After removing the slides from the solution, they were immersed in an electrophoretic chamber containing a running buffer (300 mM NaOH, 1 mM EDTA, pH>13). After 10 minutes, the slides were subjected to electrophoresis with an electricity of 24V. After a separation of about 20 minutes, the slides were transferred to a neutralization buffer (0.4 M Tris-HCl, pH 7.5) for at least 10 minutes. After drying the slides, in order to highlight nuclei fluorescence, the cells in agar were treated with a solution of 10 µg/ml ethidium bromide. Then, the cells were covered with cover-glass slides to be observed under a fluorescence microscope.

### EXAMPLE 6 (not according to the present invention).

*Analysis of antioxidant activity of tomato cell culture extracts containing SsSOD and ApSOD on tuna slices.*

Thanks to their properties of greater resistance to high temperatures and high salt concentrations, the tomato cell cultures extracts containing SsSOD and ApSOD were used in experiments on tuna slices, in order to assess their potential use as natural antioxidant additives for food products, especially for those with rapid perishability.

The tuna slices were injected with aqueous solutions of tomato cell extract containing ApSOD and tomato cell extract containing SsSOD (both at the concentration of 100 mg of dry extract per L of distilled water), while the control sample was treated with wild-type tomato cell culture extract. Subsequently, the slices were exposed for 8 days to air and light.

On the samples, colorimetric measurements were performed, which were expressed according to the CieLab method in terms of brightness (L), red index (a*) and yellow index (b*), which is considered a parameter useful for evaluating quality and freshness in fish products. As shown in the photos reported in **Figure 12****,** the slices treated with the extracts derived from the lines SsSOD#1 and ApSOD#2 had preserved their colour and brightness even after 8 days, compared to the tuna slice treated with the extract deriving from the WT line,. Both the brightness and the colorimetric parameters remained almost unchanged, while in the control slice there was a significant reduction of both parameters.

Furthermore, the histamine levels on the tuna slices were also determined. The data obtained showed that the histamine content of the tuna slices subjected to treatment with the extracts enriched with the extremophile SODs was not increased compared to the initial time (time 0). These results indicated that the extracts containing SsSOD and ApSOD could be applied in the canning industry, because they were particularly suitable for preventing damage caused by oxidation in fresh foods without causing alteration of the organoleptic properties.

### EXAMPLE 7.

*Characterization of the mixture of tomato cell culture extracts containing extremophile superoxide dismutases.*

The extracts obtained according to the examples described in Examples 1, 2 and 3 were mixed in a 1:1:1 ratio. Antioxidant activity assays were performed on the mixture at different temperature conditions, exposure to UVB rays and acidity.

**Figure 13** indicated that the mixture of the extracts (MIX) retained its enzymatic activity even after exposure to heat at a temperature of 90°C, without undergoing significant reductions; instead, the WT extract showed a reduction of the antioxidant activity by 60%. This result was attributable in particular to the action of ApSODs and SsSODs, which were hyperthermophilous, as shown by the Figures 3 and 6.

Also the effect of acid pH (pH 1.0) on the MIX did not determine any change in the activity, in contrast with what was observed for the WT wherein the activity was completely abolished at this pH value **(****Figure 14****).** This behavior was mainly attributable to the presence of the SsSOD in the MIX, which was extremely active at pH 1.0, as already reported in Figure 3. Finally, the results shown in **Figure 15** refered to the treatment of the MIX and the WT with UVB rays for 7 hours: the MIX, unlike the WT, showed a high resistance to UVB rays exposure, thanks to the presence of the enzyme DrSOD, which was active and stable in these conditions as shown in Figure 9.

### Methods.

### Analysis of enzymatic activity of the mixture (MIX) of tomato cell culture extracts containing the extremophile superoxide dismutase.

The antioxidant activity of the mixture of cell culture extracts (MIX) was determined by incubation with the NBT/Riboflavin solution, after electrophoretic run on 10% polyacrylamide gel.

12 µg of total proteins of the extract obtained from the untransformed tomato cells (WT) and 12 µg of the mixture (MIX), containing the three extracts obtained respectively from the tomato cultures containing SsSOD (4 µg), ApSOD (4 µg) and DrSOD (4 µg), were subjected to the following treatments: i) pre-incubation at 90°C for 15 minutes; ii) pre-incubation in glycine-HCl pH 1.0 buffer for 15 minutes; iii) exposure to UVB rays for 7 hours. At the end of each treatment, the samples were subjected to electrophoretic run in non-denaturing conditions followed by SOD activity staining, carried out with the NBT/Riboflavin method (as described in Example 1).

### Bibliography

Apone F, Barbulova A, Zappelli C, Colucci G (2017). Green biotechnology in cosmetics: using plant cell cultures as sources of active ingredients. HPC Today, vol. 12 (3), May/June 2017.
Ayala A, Munoz F and Arguelles S (2014). Lipid Peroxidation: Production, Metabolism, and Signaling Mechanisms of Malondialdehyde and 4-Hydroxy-2-Nonenal. Oxidative Medicine and Cellular Longevity, Vol. 2014, Article ID 360438.
Barbulova, A, Apone F. & G. Colucci (2014). Plant Cell Cultures as Source of Cosmetic Active Ingredients. Cosmetics, 1: 94-104.
Beauchamp C, Fridovich I (1971). Superoxide dismutase: improved assays and an assay applicable to acrylamide gels. Anal Biochem. 44 (1): 276-87.
Bimonte M, Tito A, Carola A, Barbulova A, Hill J, Cucchiara M, Apone F 8v Colucci G (2013). Dolichos cell culture extract for protection against UV damage. Cosmet. Toilet. 2014, 129, 46-56.
Donnelly JK, McLellan KM, Walker JL & Robinson DS (1989). Superoxide Dismutases in Foods. Food Chemistry 33: 243-270.
Ighodaro OM & Akinloye OA (2017). First line defence antioxidants-superoxide dismutase (SOD), catalase (CAT) and glutathione peroxidase (GPX): Their fundamental role in the entire antioxidant defence grid. Alexandria Journal of Medicine.
https://doi.org/10.1016/j.ajme.2017.09.001.
Noble JE (2014). Quantification of protein concentration using UV absorbance and Coomassie dyes. Methods in Enzymology, 536: 17-26.
Tito A, Carola A, Bimonte M, Barbulova A, Arciello S, De Laurentiis F, Monoli I, Hill J, Gibertoni S, Colucci G & Apone F (2011). A tomato stem cell extract, containing antioxidant compounds and metal chelating factors, protects skin cells from heavy metal induced damages. Int. J. Cosmet. Sci., 33(6): 543-552.
Valgimigli L, Sapone A, Canistro D, Broccoli M, Gatta L, Soleti A, Paolini M (2015). Oxidative stress and aging: a non-invasive EPR investigation in human volunteers. Aging Clin Exp Res. 27(2): 235-8.

## Claims

1. A composition with antioxidant activity comprising a first dry extract derived from plant cell cultures comprising superoxide dismutase of *Sulfolobus solfataricus* expressed in said plant cells, a second dry extract derived from plant cell cultures comprising superoxide dismutase of *Aeropyrum pemix* expressed in said plant cells, and a third dry extract derived from plant cell cultures comprising superoxide dismutase of *Deinococcus radiodurans* expressed in said plant cells.

2. The composition according to claim 1, wherein said plant cell cultures are cultures of cells belonging to *Solanum lycopersicum* species.

3. The composition according to claim 1 or 2, containing, for one part by weight of said dry extract comprising superoxide dismutase of *Sulfolobus solfataricus*, from 0.01 to 100, preferably 0.5-2, parts by weight of said dry extract comprising superoxide dismutase of *Aeropyrum pernix* and, independently, from 0.01 to 100, preferably 0.5-2, parts by weight of said dry extract comprising superoxide dismutase of *Deinococcus radiodurans.*

4. The composition according to claim 3, containing, for one part by weight of said dry extract comprising superoxide dismutase of *Sulfolobus solfataricus*, one part by weight of said dry extract comprising superoxide dismutase of *Aeropyrum pernix* and one part by weight of said dry extract comprising superoxide dismutase of *Deinococcus radiodurans.*

5. The composition according to any one of the preceding claims, further comprising at least one hydrophilic solvent selected from the group comprising water, aqueous saline solutions and organic solvents.

6. Cosmetic use of a composition according to any one of claims 1-5.

7. Use of the composition according to any one of claims 1-5 as food preservative.

8. Cosmetic formulation for topical application, comprising a composition according to any one of claims 1-5, at least one cosmetically active substance and a cosmetically acceptable vehicle.

9. The cosmetic formulation according to claim 8, wherein said formulation is a gel, a cream or a lotion.

10. A food or nutraceutical product comprising a composition according to any one of claims 1-5.

## Patentansprüche

1. Zusammensetzung mit antioxidativer Aktivität, umfassend einen ersten Trockenextrakt aus Pflanzenzellkulturen, umfassend Superoxid-Dismutase von *Sulfolobus solfataricus*, die in den Pflanzenzellen exprimiert ist, einen zweiten Trockenextrakt aus Pflanzenzellkulturen, umfassend Superoxid-Dismutase *von Aeropyrum pernix*, die in den Pflanzenzellen exprimiert ist, und einen dritten Trockenextrakt aus Pflanzenzellkulturen, umfassend Superoxid-Dismutase von *Deinococcus radiodurans*, die in den Pflanzenzellen exprimiert ist.

2. Zusammensetzung gemäß Anspruch 1, wobei die Pflanzenzellkulturen Kulturen von Zellen sind, die zur Spezies *Solanum lycopersicum* gehören.

3. Zusammensetzung gemäß Anspruch 1 oder 2, enthaltend für einen Gewichtsanteil des Trockenextrakts, der Superoxid-Dismutase von *Sulfolobus solfataricus* umfasst, von 0.01 bis 100, vorzugsweise 0.5-2 Gewichtsanteile des Trockenextrakts, der Superoxid-Dismutase von *Aeropyrum pernix* umfasst, und, unabhängig davon, von 0.01 bis 100, vorzugsweise 0.5-2 Gewichtsanteile des Trockenextrakts, der Superoxid-Dismutase von *Deinococcus radiodurans* umfasst.

4. Zusammensetzung gemäß Anspruch 3, enthaltend für einen Gewichtsanteil des Trockenextrakts, der Superoxid-Dismutase von *Sulfolobus solfataricus* umfasst, einen Gewichtsanteil des Trockenextrakts, der Superoxid-Dismutase von *Aeropyrum pernix* umfasst und einen Gewichtsanteil des Trockenextrakts, der Superoxid-Dismutase von *Deinococcus radiodurans* umfasst.

5. Zusammensetzung gemäß einem der vorhergehenden Ansprüche, ferner umfassend mindestens ein hydrophiles Lösungsmittel, ausgewählt aus der Gruppe umfassend Wasser, wässrige Salzlösungen und organische Lösungsmittel.

6. Kosmetische Verwendung einer Zusammensetzung gemäß einem der Ansprüche 1-5.

7. Verwendung der Zusammensetzung gemäß einem der Ansprüche 1-5 als Konservierungsmittel für Lebensmittel.

8. Kosmetische Rezeptur zur topischen Anwendung, umfassend eine Zusammensetzung gemäß einem der Ansprüche 1-5, mindestens eine kosmetisch aktive Substanz und einen kosmetisch verträglichen Trägerstoff.

9. Kosmetische Rezeptur gemäß Anspruch 8, wobei die Rezeptur ein Gel, eine Creme oder eine Lotion ist.

10. Nahrungsmittel oder nutrazeutisches Produkt, umfassend eine Zusammensetzung gemäß einem der Ansprüche 1-5.

## Revendications

1. Composition avec activité antioxydante comprenant un premier extrait sec dérivé de cultures de cellules de plante comprenant une superoxyde dismutase de *Sulfolobus solfataricus* exprimée dans lesdites cellules de plante, un second extrait sec dérivé de cultures de cellules de plante comprenant une superoxyde dismutase d'*Aeropyrum pernix* exprimée dans lesdites cellules de plante, et un troisième extrait sec dérivé de cultures de cellules de plante comprenant une superoxyde dismutase de *Deinococcus radiodurans* exprimée dans lesdites cellules de plante.

2. Composition selon la revendication 1, dans laquelle lesdites cultures de cellules de plante sont des cultures de cellules appartenant aux espèces *Solanum lycopersicum.*

3. Composition selon la revendication 1 ou 2, contenant, pour une partie en masse dudit extrait sec comprenant une superoxyde dismutase de *Sulfolobus solfataricus,* de 0,01 à 100, de préférence 0,5-2, parties en masse dudit extrait sec comprenant une superxoyde dismutase d'*Aeropyrum pernix* et, indépendamment, de 0,01 à 100, de préférence 0,5-2, parties en masse dudit extrait sec comprenant une superoxyde dismutase de *Deinococcus radiodurans.*

4. Composition selon la revendication 3, contenant, pour une partie en masse dudit extrait sec comprenant une superoxyde dismutase de *Sulfolobus solfataricus*, une partie en masse dudit extrait sec comprenant une superoxyde dismutase d'*Aeropyrum pernix* et une partie en masse dudit extrait sec comprenant une superoxyde dismutase de *Deinococcus radiodurans.*

5. Composition selon l'une quelconque des revendications précédentes, comprenant de plus au moins un solvant hydrophile choisi dans le groupe comprenant de l'eau, des solutions salines aqueuses et des solvants organiques.

6. Utilisation cosmétique d'une composition selon l'une quelconque des revendications 1-5.

7. Utilisation de la composition selon l'une quelconque des revendications 1-5 comme un conservateur alimentaire.

8. Formulation cosmétique pour application topique, comprenant une composition selon l'une quelconque des revendications 1-5, au moins une substance cosmétiquement active, et un véhicule cosmétiquement acceptable.

9. Formulation cosmétique selon la revendication 8, dans laquelle ladite formulation est un gel, une crème ou une lotion.

10. Produit alimentaire ou nutraceutique comprenant une composition selon l'une quelconque des revendications 1-5.
